# EUROPEAN PATENT SPECIFICATION

(11) **EP 1 438 587 B1**
(45) Date of publication and mention of the grant of the patent: **30.07.2008**
(21) Application number: 02763668.7
(22) Date of filing: 18.09.2002
(51) Int. Cl.: G01N 33/58

(54) **SYSTEM FOR THE DETECTION OF UREASE AND METHOD FOR USING SAME**
VORRICHTUNG UND VERFAHREN ZUR ERKENNUNG VON UREASE
SYSTEME DE DETECTION D'UREASE ET PROCEDE D'UTILISATION CORRESPONDANT

(30) Priority: 15.10.2001 US 977555; 15.10.2001 US 977556; 15.10.2001 US 977874; 15.10.2001 US 977667
(43) Date of publication of application: 21.07.2004
(60) Divisional application: 08000375.9
(73) Proprietor: Marshall, Barry, Subiaco 6904 (AU)
(72) Inventor: MCMICHAEL, Donald, J., South Jordan, UT 84095 (US); PETERSON, Kristy, Salt Lake City, UT 84117 (US); MARSHALL, Barry, J., Dalkeith, Western Australia 6009 (AU); MENDIS, Aruni, H., W., Connolly, Western Australia 6027 (AU); CHAIRMAN, Simon, Beaconsfield, Victoria 3807 (AU)
(74) Representative: Grünecker, Kinkeldey, Stockmair & Schwanhäusser Anwaltssozietät
(86) International application number: PCT/US2002/029814
(87) International publication number: WO 2003/034061

(56) References cited:
- EP-A- 0 896 547
- US-A- 4 748 113
- US-A- 5 782 951

## Description

### Background of the Invention

Many ailments of the gastrointestinal system in humans are caused at least in part by bacteria. Such bacteria include those of the genus *Campylobacter,* and particularly *Helicobacter pylori.* For example, *Helicobacter pylori* can cause bacterial infections on the mucosal surface of the gastrointestinal tract, particularly on the surface of the stomach. The chronic disorders of the gastrointestinal system that can be caused by bacteria Include chronic or atrophic gastritis, gastroenteritis, non-ulcer dyspepsia, esophageal reflux disease, gastric motility disorders, peptic ulcers including gastric and duodenal ulcers, and the like.

Once a patient is showing symptoms of a gastrointestinal disorder, several tests can be used to diagnose the disorder, including the diagnosis of a possible bacterial Infection. Diagnostic testing systems have been manufactured to test for a wide variety of conditions in numerous types of samples, such as, for example, blood, tissue biopsies, and saliva. Such testing systems may be utilized to determine the presence of particular bacteria, such as *Helicobacter pylori.* Some tests that have been proposed to detect *Helicobacter pylori* include those that are disclosed in numerous U.S. Patents, including, for example, U.S. Patent No. 4,748,113 to Marshall, U.S. Patent No. 5,314,804 to Boguslaski et al., U.S. Patent No. 5,439,801 to Jackson, U.S. Patent No. 5,702,911 to Whalen, U.S. Parent No. 5,989,840 to D'Angelo et al., U.S. Patent No. 6,068,985 to Cripps et al., U.S. Patent No. 6,156,346 to Chen et al., and U.S. Patent No. 6,187,556 to Lee et al.

*Helicobacter pylori* produces an enzyme called urease. Various tests detect the presence of urease on a sample, such as, for example, a gastric sample that is obtained through endoscopy. In the tests described above, other biological samples may be used, such as, for example, blood, saliva, or urine. Urease is known to convert urea into ammonium carbonate, which then decomposes into ammonia and carbon dioxide. Consequently, in the past, one test for detecting the presence of *Helicobacter pylori* included the steps of contacting a sample of gastric material with a composition containing urea and an indicator, namely a pH indicator that changes color when there is a rise in pH. If urease is present within the gastric material it breaks down the urea, which results in the formation of ammonia after further decomposition and causes the pH indicator to change color.

The gastric material that is collected from the patient is typically a biopsy specimen that is removed from the gastric mucosa at endoscopy by means of biopsy forceps. Typically, the tissue sample is inserted into a gel that contains urea and the indicator.

Although the above method has provided great advancements in the early detection of gastrointestinal disorders, the testing composition used to detect the presence of urease has a limited shelf life. In particular, the urea and other reagents contained within the composition can have a tendency to degrade over time. Consequently, once formulated, the testing composition should be used in a relatively short amount of time and is also typically refrigerated prior to use in order to prevent degradation.

In view of the above, a need currently exists for an improved testing composition and associated method for the detection of bacterial infections in the gastrointestinal tract of patients. More particularly, a need exists for a composition for detecting urease in gastric samples that has a prolonged shelf life.

### Summary of the Invention

The present invention is directed to further improvements in the detection of bacterial infections in the gastrointestinal tract. In one embodiment, for instance, the present invention is directed to a system for detecting the presence of urease in a gastric sample in order to indicate the presence of *Helicobacter pylori.* The system includes a first composition that is maintained separate from a second composition for sequential contact with the sample. The first composition includes urea in a dried and finely powdered state. The urea is capable of being converted into ammonia when contacted with urease. The powdered urea can have a mean particle size of less than about 0.1 mm, and particularly less than about 0.05 mm. Besides urea, the first composition can also include other powder-like components, such as an anti-caking agent to prevent the fine urea from clumping or "caking".

The second composition, on the other hand, can contain an indicator and can be configured to indicate the presence of ammonia. For instance, the indicator can be a pH indicator that changes color when the pH of the second composition is increased to a certain level. For example, the indicator can be phenol red, which changes from yellow to red when exposed to a pH of greater than about 6.8.

In accordance with the present invention, the gastric biopsy sample is grasped, such as with a specimen-handling tool and is then contacted with the first composition. The urea powder contacts and sticks to the gastric biopsy sample. Should the gastric sample contain urease, the urea is converted into ammonia.

After contacting the first composition, the gastric material is then contacted with the second composition containing the indicator. The indicator indicates the presence of ammonia that, in turn, is a positive test for the detection of urease.

By maintaining the first composition containing urea separate from the second composition containing an indicator, various advantages and benefits are realized. In particular, the urea remains more stable and therefore the system has an increased shelf life. Further, the gel mixture is stable during manufacture so that much larger and longer duration production runs can be made without concern for slight temperature variations during the process.

Besides containing an indicator, the second composition can contain various other ingredients. For example, the second composition can be in a gel-like state and can contain a gel, such as agar. To maintain a low pH in the second composition within desired limits, the second composition can also contain a pH adjuster, such as an acid or buffering agent. For example, in one embodiment, the pH adjuster can maintain the pH of the second composition in a range of from about 4.5 to about 6. The second composition can also contain a bactericide, which can inhibit the growth of other organisms.

The first and second compositions can be contained within separate containers or can be spaced apart in the same container. For example, in one embodiment, a container can be used that includes a first well and a second well. The first composition can be located in the first well, while the second composition can be located in the second well. The container can be made from plastic and can include an overlying member, such as a peelable top made from a film. The overlying member may be positioned over at least a portion of one or more of the wells and/or the cavity. The film can be substantially or completely water impermeable over the first well to prevent any moisture from contacting the urea.

A specimen-handling tool may be disposed about at least a portion of one of the wells. The specimen-handling tool may be disposed within a cavity formed in the container. The specimen-handling tool may be adapted to manipulate a specimen such as a biopsy sample.

The specimen-handling tool may include a pair of cooperating arms. Each arm may include a tip portion and a rear portion, the arms being joined to each other at their rear portions. Each arm may further include a rearward arcuate portion, a forward arcuate portion, and an intermediate arcuate portion that is disposed between the rearward arcuate portion and the forward arcuate portion. The arcuate portions may be configured so that the area disposed between the pair of arms is approximately hourglass in shape.

Other features and advantages of the present invention will be discussed in greater detail below.

### Brief Description of the Drawings

A full and enabling disclosure of the present invention, including the best mode thereof, to one of ordinary skill in the art is set forth more particularly in the remainder of the specification, including reference to the accompanying figures, in which:
Figure 1 is a perspective view of one embodiment of a system for detecting urease in accordance with the present invention;
Figure 2 is a top view of the system illustrated in Figure 1;
Figure 3 is a cross-sectional view of the system illustrated in Figure 1;
Figure 4 is a cross-sectional view of an embodiment of a urease testing device.
Figure 5 is a perspective view of an embodiment of the system, container and specimen-handling tool of the present invention;
Figure 6 is a perspective view of an embodiment of the container of the present invention;
Figure 7 is a perspective view of the bottom of an embodiment of the container of the present invention;
Figure 8 is a side view of an embodiment, of the container of the present, invention;
Figure 9 is a top view of another embodiment of the container of the present invention;
Figure 10 is a perspective view of an embodiment of the specimen-handling tool of the present invention;
Figure 11 is a side view of an embodiment of the specimen-handling tool of the present invention depicted in Figure 10;
Figure 12 is another perspective view of an embodiment of the specimen-handling tool of the present invention;
Figure 13 is a top view of an embodiment of the specimen-handling tool of the present invention that is depicted in Figure 12;
Figure 14 is a perspective view of yet another embodiment of the specimen-handling tool of the present invention;
Figure 15 is a perspective view of still another embodiment of the specimen-handling tool of the present invention;
Figure 16 is a perspective view of another embodiment of the system, carrier and specimen-handling tool of the present invention;
Figure 17 is a cross-sectional view of the embodiment depicted in Figure 16, taken along line 13-13;
Figure 18 is a perspective cross-sectional view of the embodiment depicted in Figure 16, taken along line 14-14;
Figure 19 is a perspective view of an embodiment of the system of the present invention;
Figure 20 is a cross-sectional view of the embodiment depicted in Figure 18, taken along line 16-16; and
Figure 21 is a perspective view of another embodiment of the specimen-handling tool of the present invention.

Repeated use of reference characters in the present specification and drawings is intended to represent same or analogous features or elements of the invention.

### Detailed Description of Embodiments

Reference will now be made in detail to the embodiments of the invention, one or more examples of which are set forth below. Each example is provided by way of explanation, not limitation of the invention. For instance, features illustrated or described as part of one embodiment, can be used on another embodiment to yield a still further embodiment. Thus, it is intended that the present invention covers such modifications and variations as come within the scope of the appended claims.

The present invention is generally directed to a system and method for the detection of gastrointestinal disorders caused by bacterial infections. More particularly, the system and method of the present invention detect the presence of urease on a gastric biopsy sample. Urease is an enzyme known to be produced by bacteria that are harmful to the gastrointestinal tract, including bacteria such as *Helicobacter pylori.* Gastrointestinal disorders that can be caused by bacterial infections include chronic or atrophic gastritis, gastroenteritis, non-ulcer dyspepsia, esophageal reflux disease, gastric motility disorders, peptic ulcers including gastric and duodenal ulcers, and the like.

In the past, in order to detect bacterial infections in the gastrointestinal, tract, a biopsy sample of gastric material was first obtained. The biopsy sample was then contacted with a composition containing urea and an indicator, such as a pH indicator. If urease were present in the biopsy sample, the urease would break down and convert the urea in the composition to ammonia subsequently causing a rise in the pH of the composition. The rise in pH then caused the indicator to undergo a color change.

As described above, however, the composition containing urea and the indicator has a relatively short shelf life due to the instability of various ingredients in the composition, including the urea. The present invention is directed to an improved test for gastrointestinal disorders caused by bacterial infections. According to the present invention, in order to improve the shelf life of systems and devices designed to detect bacterial infections in the gastrointestinal tract, a composition containing urea is separated from a composition containing an indicator. The two compositions are then sequentially contacted with a biopsy sample in order to detect the presence of urease.

More particularly, the first composition contains urea in a finely powdered, dry state. By maintaining urea in a powdered form separate from the agar and the indicator, the urea remains more stable. Further, by maintaining the urea separate from the indicator, the handling requirements of the test system become more relaxed. For instance, by maintaining both compositions separate, there is no need to refrigerate the compositions prior to use or during shipping.

By maintaining the urea separate from the indicator composition, the process conditions for manufacturing the indicator composition also become relaxed. In particular, the indicator composition, such as an indicator gel, is much more stable during manufacture, allowing larger batches to be produced that are not sensitive to ingredients contained within the composition and to temperature variations.

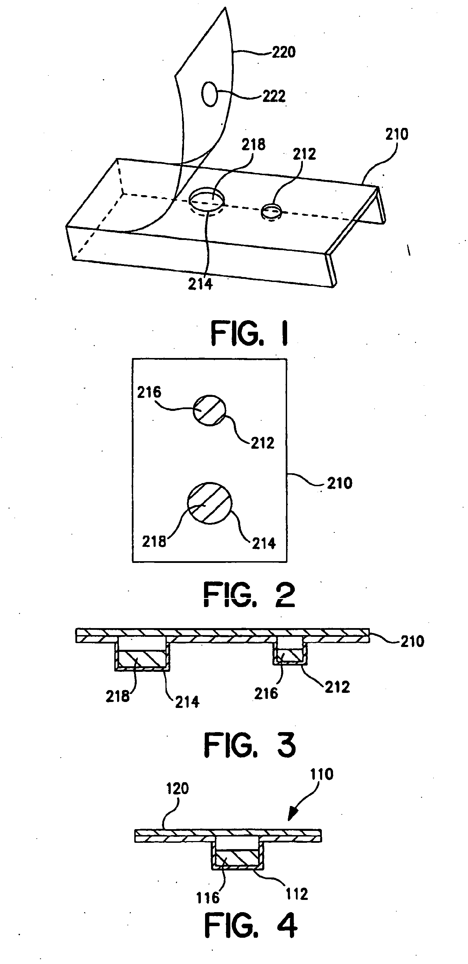

The system of the present invention for detecting urease can come in many forms, and for purposes of explanation Figures 1-3 illustrate another embodiment of a device for detecting urease in biopsy samples in accordance with the present invention. As shown, the testing device in this embodiment includes a single container 210 defining a first well 212 and a second well 214. Contained in the first well 212 is a first composition 216 containing urea, such as urea in a finely powdered state with or without an anti-caking agent.

In the second well 214, on the other hand, is a second composition 218 containing an indicator. The indicator is configured to detect the presence of ammonia.

In this embodiment, the device 210 further includes a removable top 220 that covers the first well 212 and the second well 214. For example, the top 220 can be made from a plastic film. The top 220 is provided in order to prevent the first composition 216 or the second composition 218 from spilling or becoming contaminated prior to use.

In order to protect the powdered urea, the film top 220 can be made liquid impermeable at a location over the first well 212. In particular, the entire film top 220 can be liquid impermeable or, alternatively, a separate membrane 222 as shown in Figure 1 can be placed over the first well 212 that is liquid impermeable.

Besides or in addition to making the top film 220 liquid impermeable, the membrane 222 can also be used to prevent urea particles from sticking to the film top when the film top is removed.

In order to perform a urease test using the device shown in Figure 1, a biopsy sample is first taken from the lining of the gastrointestinal tract of a patient, such as from the lining of the stomach. The biopsy sample can be taken at endoscopy using biopsy forceps. The top film 220 is peeled back to expose the first composition 216 in the first well 212. The biopsy sample is then contacted with the first composition causing the urea powder to stick to the sample. For example, the biopsy sample can be rolled in the first composition much like the process of "flouring" a food product prior to cooking.

Once the first composition has coated the biopsy sample, the sample is then contacted with the second composition 218 containing an indicator located in the second well 214. Once contacted with the second composition, the powdered urea on the surface of the biopsy sample is moistened and activated by the second composition. Once moistened, the urea powder becomes available in greater amounts to any urease enzyme present in the biopsy sample. If present, the urease converts the urea into the unstable ammonium bicarbonate, which further decomposes into ammonia and carbon dioxide. The indicator present in the second composition indicates the presence of ammonia to signify a positive test for urease. For example, in one embodiment the indicator can be a pH indicator that changes color when the pH of its environment is increased.

Besides both compositions being spaced apart on a single container or platform as shown in Figure 1, however, it should be understood that the first and second compositions of the present invention can be maintained in any suitable separated state prior to testing. In this regard, the first composition and the second composition can be maintained in separate containers if desired.

The ingredients that can be contained in the first composition and the second composition in accordance with the present invention will now be described in greater detail. As described above, the first composition is generally a dry or moisture-free composition containing urea in a powdered state. Urea has the chemical formula H₂NCONH₂ and is a naturally occurring product of protein metabolism. When contacted with urease, urea hydrolyzes to form unstable ammonium bicarbonate, which further decomposes into ammonia and carbon dioxide.

Urea in a powdered state for use in the present invention is available from various commercial sources. The particle size of the urea contained in the first composition is generally not critical although smaller particle sizes work more efficiently. In this regard, if desired, the urea can be ground to have a mean particle size of less than about 0.1 mm, particularly less than 0.05 mm, and more particularly less than about 0.01 mm. It should be understood, however, that even smaller particle sizes may be used. For example, in one embodiment, the urea particles can have a mean particle size of less than 3 microns, and particularly less than 1 micron. By reducing the particle size, more surface area of urea is available for reaction with urease and the urea will better stick to the biopsy sample.

When using relatively smaller particles, the urea particles can have a particle size distribution such that no particles present have a size greater than about 100 microns, particularly no greater than about 10 microns, and more particularly no greater than about 5 microns. The particle size of the urea can be determined using any suitable method, such as by using transmission electron microscopy (TEM). When using transmission electron microscopy, the average diameter of each particle is measured, followed by calculating the mean diameter of the urea particles in a particular group. The average diameter of each particle can be calculated by taking the average of the smallest diameter of the particle and the largest diameter of the particle. Besides transmission electron microscopy, light scattering can also be used to determine particle sizes. The mean particle size of the urea particles in a particular group is calculated by adding the sizes of the particles together and dividing by the number of particles.

Besides containing urea, the first composition can also contain various other dry additives. For example, in one embodiment, if desired, an anti-caking agent can also be contained within the first composition. The anti-caking agent will prevent the fine urea powderfrom clumping or "caking". Any suitable anti-caking agent can be used in the present invention. For example, in one embodiment, fine silicon dioxide or fine sodium alumino silicate powder can be contained in the first composition. The weight per weight (w/w) ratio of urea/silicon dioxide contained in the first composition can be any ratio from 1/1 to 100/1. The particle size of the anti-caking agent can vary depending upon the particular application. For instance, in one embodiment, the particle size of the anti-caking agent is no greater than the particle size of the urea.

The second composition, which is maintained separate from the first composition, contains an indicator for indicating the presence of ammonia. In general, any suitable indicator can be present in the second composition. In one embodiment, a pH indicator can be used that indicates a change in pH. For example, various pH indicators are available that change color as the pH is increased.

In general, when using a pH indicator, the pH of the second composition should be less than about 6.5. More particularly, the second composition can have a pH that is consistent with mammalian tissue, which typically has a pH of about 6.5.

In this regard, the pH of the second composition should be from 4.0 to 6.5, and particularly from about 4.5 to about 6.0. In this manner, when the second composition is contacted with the biopsy sample containing urea, the pH of the second composition will increase if the urea is being converted into ammonia. This rise in pH will then cause the pH indicator to signify a positive reading, such as by changing color.

The pH of the second composition should be adjusted to have a pH of from about 0.5 pH unit to about 2 pH units lower than that necessary for a color change to occur.

Consequently, when using a pH indicator, the indicator should undergo a color change or otherwise signify a positive reading when the pH of the second composition rises above neutral, and particularly above about 7.5. pH indicators useful in the present invention include indicators that undergo a change in color over a pH range of from about 5.5 to about 9.0, and particularly from about 6.5 to about 8.5.

One particular pH indicator that can be used in the present invention is phenol red. Phenol red changes from a yellow color to a red color as the pH of its surroundings increase. Phenol red is also referred to as phenolsulfonphthalein.

Other pH indicators that may be used in the present invention include p-nitro-phenol, bromthymol blue (dibromthymolsulfonph-thalein), neutral red (2-methyl-3-amino-6-dimethylaminophenazine), quino-line blue (cyanine), cresol red (o-cresolsulfonphthalein), matacresol purple (m-cresolsulfonphthalein), thymol blue (thymolsulfonphthalein), bromocresol purple (4,4'-(3H-2,1-benzoxathiol-3-ylidene)bis[2-bromo-6-methylphenol] S,S-dioxide), chlorophenol red, bromocresol green (4,4'-(3H-2,1-benzoxathiol-3-ylidene)bis[2,6-dibromo-3-methylphenol] S,S-dioxide), and bromophenol blue (4,4'-(3H-2,1-benzoxathiol-3-ylidene)bis[2,6-dibromophenol] S.S-dioxide).

In one embodiment, a combination of indicators can be used, such as described in U.S. Patent No. 5,439,801 to Jackson. For example, in one embodiment, methyl red can be combined with bromthymol blue.

The second composition can be made up entirely of the indicator or can include other ingredients as desired. For example, in one embodiment, the indicator can be present in a gel-like material. In this regard, the indicator can be combined with a gelling agent so that the second composition is In a semi-solid state under ambient conditions.

In one embodiment, the gelling agent can be agar. Agar is a polysaccharide complex that is extracted from agarocytes of certain algae. Agar is available from various commercial sources. For most applications, the agar or any other gelling agent used should be nonnutritive, i.e., does not support the growth of microorganisms.

Besides, or in addition to, a gelling agent, an indicator can also be combined with a pH adjuster to maintain the pH of the second composition within preset limits. The addition of a pH adjuster is particularly beneficial when using a pH indicator to prevent against false readings. For example, as discussed above, the pH of the second composition should be from about 4.0 to about 6.5, when using a pH indicator. A suitable pH adjuster can be used to maintain the pH of the composition within this range. pH adjusters suitable for this test include acids and buffering agents. The use of a pH adjuster depends upon the make-up of the second composition and the requirements of the test. For example, reduction of the amount of buffer in the second composition leads to a much faster reaction and a faster change in colors by the indicator, with the most rapid reaction rate occurring in the absence of a buffering agent, as compared to the reaction rate when using a large amount of a buffering agent. Thus, if a high reaction rate is required, the use of buffering agents as pH adjusters should be limited.

In general, any suitable pH adjuster can be used, depending upon the requirements of the test and the second composition, including the use of acids and buffering agents such as sodium citrate, phosphate-citrate, citric acid, sulfamic acid, sodium bisulfate, sodium acetate, sodium phosphate, and potassium phosphate.

Another ingredient that may be contained in the second composition is a bactericide or a bacteristat. The bactericide or bacteristat can be used to act as a preservative for any of the other ingredients or can be used to substantially inhibit the growth of other organisms to prevent against false readings. Bactericides that can be used in the present invention include sodium azide, methyl paraben (methyl p-hydroxybenzoate), and propyl paraben (propyl p-hydroxybenzoate).

The amount of each ingredient added to the second composition will depend upon the various circumstances and the desired result. Besides maintaining the indicator as a liquid or in a gel state, the indicator can also be contained in an absorbent substrate, such as a substrate made from pulp fibers including cardboard or paper. In this embodiment, the second composition can be dry and relatively moisture free. When using a paper substrate, however, extra water and distilled water, may need to be added to the second composition in combination with the biopsy sample in order to provide enough moisture to activate the indicator.

The following is an example of one formulation that can be used as the second composition in the system of the present invention. The pH of the solid gel will be between 4 and 6.5 and particularly between 4.5 and 6.0.

| **Ingredient** | **Amount** |
|---|---|
| Agar (Extra Pure Grade) | 1.0-50.0g |
| Citric Acid | 0.001-1.0g |
| Phenol Red | 0.001-2.0g |
| Methylhydroxy Benzoate | 0.01-100.0g |
| Distilled Water | remainder |

When forming a one liter batch of the above composition, the ingredients can be added in the following amounts.

| **Ingredient** | **Reference** | **Amount** |
|---|---|---|
| Agar (Extra Pure Grade) | Merck Catalog #1.01615.9025 | 15.0g |
| Citric Acid | Merck Catalog #1.00247.1000 | 0.0145g |
| Phenol Red | Merck Catalog #1.07241.0025 | 0.110g |
| Methyl Paraben | Merck Catalog #1.06757.5000 | 2.0g |
| Distilled Water | -- | 1000mL |

In producing the above gel composition, the distilled water is first heated to 95 °C. The phenol red powder is added while stirring the distilled water, and the agar is added in small amounts while the mixture is maintained at 95 °C. The citric acid and methyl paraben are then added to the mixture. The bulk liquid is cooled to 50 °C and dispensed in an amount of 0.2 mL into the second well of the present invention.

The first well of the container can contain 5 to 50 mg of the first composition, and optimally 30 mg of the resulting fine powder mixture. In the preparation of the urea mixture of the first composition for use in the first well, crystalline extra pure urea (Merck Catalog #1.08486.5000) is mixed with silicon dioxide (Sigma Catalog #S-5631) at a weight-to-weight ratio from 1:1 to 100:1, and in one embodiment in a weight-to-weight ratio of 2:1. The mixture is subject to grinding until a fine powder mixture results.

An embodiment useful for a better understanding of the invention is illustrated in Figure 4. In this embodiment, instead of containing two separate wells and two separate compositions, the urease testing device contains a single composition in a dry powdered state. Specifically, in this embodiment, the urease indicating composition contains dry powdered urea combined with a dry powdered indicator.

For example, as shown in Figure 4, a urease testing device generally 110 includes a single well 112 covered by a peelable plastic film 120. The well 112 includes a urease indicating composition 116, which contains a powdered mixture or urea and an indicator.

The powdered urea contained within the well can be a urea as described above having an average particle size of less than about 0.1 mm, particularly less than about 0.05 mm, and more particular less than about 0.01 mm. Combined with the powdered urea is a dry or powdered indicator, such as a pH indicator. In general, any suitable dry indicator can be present in the composition, such as any of the above- described indicators. The amount of indicator contained within the composition will generally depend upon the particular indicator chosen. Specifically, the indicator should be present in the composition in an amount sufficient to show a color change when the composition is contacted with urease present in a biopsy sample.

In this embodiment, the biopsy sample is placed in the well and mixed with the powdered composition. Any moisture present in the biopsy sample can be used to activate the urea and the indicator. If necessary, however, an aqueous solution, such as distilled water, can be added with the biopsy sample. If urease is present in the biopsy sample, the urease will convert the urea into ammonia which, in turn, will cause the indicator to indicate a positive result, such as by changing color.

If desired, an anti-caking agent as described above can also be contained in the dry powdered composition. In this embodiment, however, a pH adjuster or a bactericide will most likely not be needed, although both ingredients can be contained in the composition if desired.

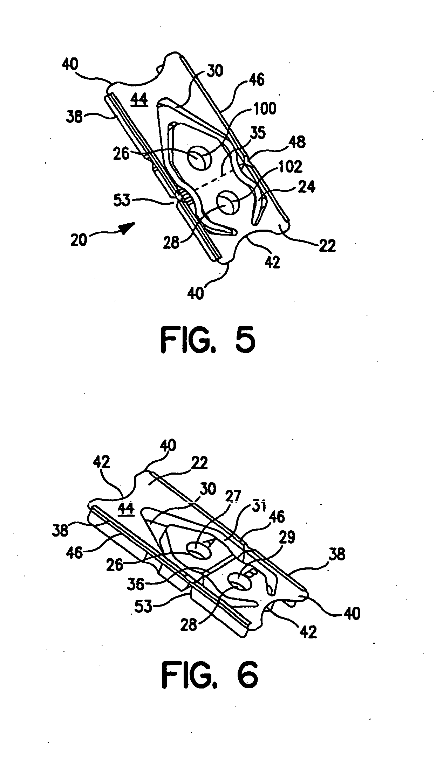

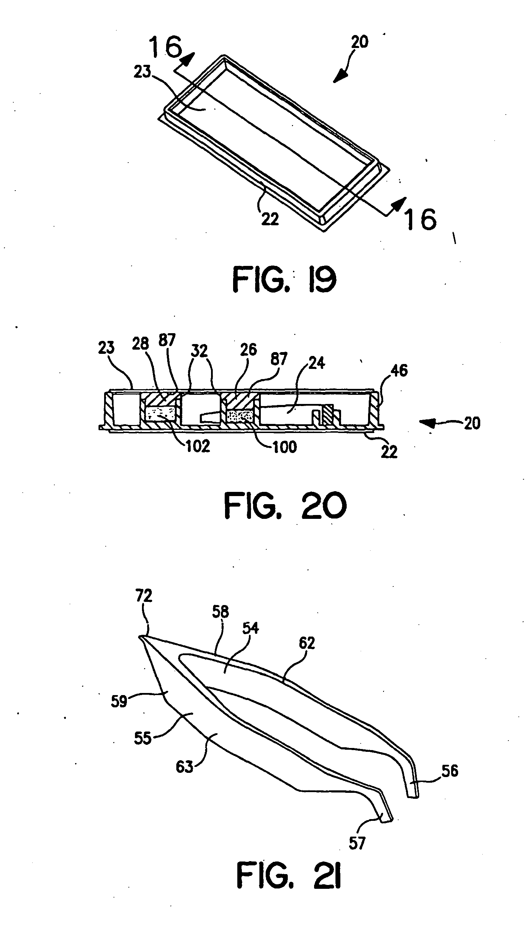

In one embodiment, the present invention is directed to containers and tools for use in the disclosed systems and methods. Figure 5 discloses an embodiment of a diagnostic system 20 according to the present invention that may be utilized for many types of diagnostic testing. Such diagnostic tests utilize a biological test specimen such as, for example, tissue biopsy, blood or saliva. The diagnostic system 20 may include a container 22 and a mechanism by which a user may manipulate a sample of tissue, such as, for example, the specimen-handling tool 24 that is shown in Figures 5, 10 and 14. As depicted in Figure 19, the diagnostic system 20 may further include an overlying member 23.

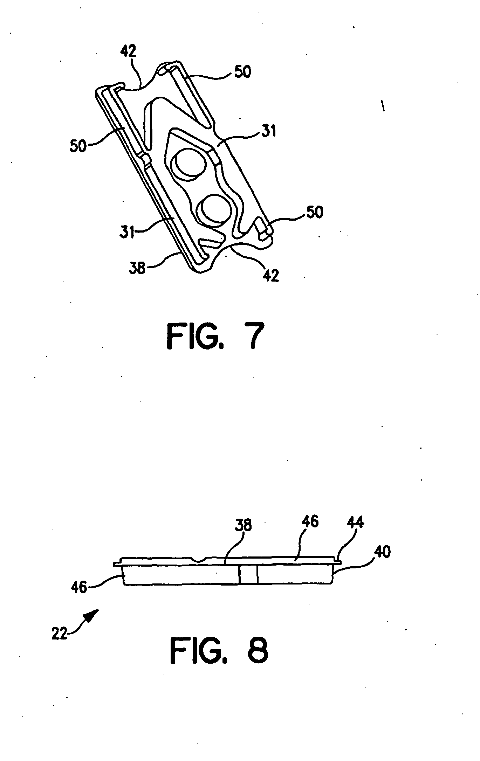

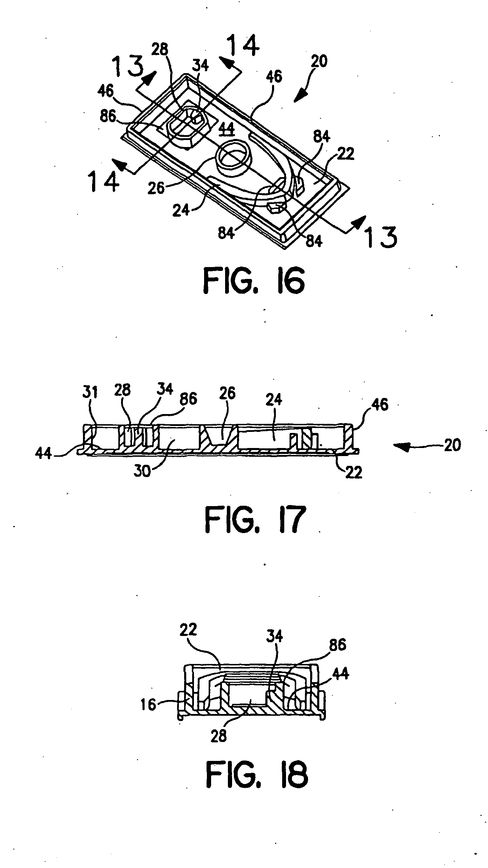

As shown in Figures 5-7, 9, and 16, the container 22 may include a first well 26 and a second well 28. The wells 26 and 28 may be defined, at least in part, by the walls 27 and 29, respectively. The wells 26 and 28 may be formed to have a variety of different depths and cross-sectional shapes, some variations of which are shown in Figures 5, 16-18 and 20. The wells 26 and 28 of the container 22 may be variously formed, and may have similar configurations or dissimilar configurations. As shown in Figures 7 and 17, the wells 26 and/or 28 may be generally frustoconical in shape, although the wells 26 and/or 28 may be cylindrical or otherwise shaped. The wells 26 and/or 28 may be formed so that, when viewed from the top of the container 22, the wells 26 and/or 28 have a non-circular shape, such as an elliptical, square, rectangular, D-shaped or any other shape.

One or more projecting members, such as the projecting member 34 that is shown in Figures 16-18, may be disposed within one or both of the wells 26 and 28. At least a portion of the projecting member 34 may be disposed outside of the interior of the wells 26 and/or 28. The projecting member 34 may be integrally formed with the walls 27 and 29, or may be attached to the walls 27 and/or 29. Such projecting members 34 may be configured to assist removal of the specimen such as, for example, a biopsy specimen, from the specimen-handling tool 24. These projecting members 34 may be configured to assist the user in accurately positioning a specimen within the well 26 or 28.

The wells 26 and 28 may also include a step such as the step 32 that is depicted in Figure 20.

The container 22 may have many different overall exterior shapes, such as, for example, the generally rectangular shape as shown in Figures 5, 6 and 9. The container 22 may be alternately shaped, such as, for example, square, oblong, triangular, and the like. The container 22 may, as shown in Figures 5-7, include two elongated sides 38, two ends 40 and a surface 44. The ends 40 may be configured to be easily grasped by a user and one, none or both of the ends 40 may include an arcuate portion 42 as shown in Figures 5 - 9.

As shown in Figures 5, 6, 8 and 9, the container 22 may include a surface 44. The first and/or second wells 26 and 28, respectively, may be configured to extend downwardly from the surface 44. As shown in Figures 5 and 6, the container 22 may also include a cavity 30. In a similar manner, the cavity 30 may be configured to extend downwardly from the surface 44, as shown in Figures 5, 6 and 9. As shown in Figures 16-18, one or both of the wells 26 and 28 and/or the cavity 30 may be formed so as to extend upwardly from at least a portion of the surface 44.

A mechanism by which a user may manipulate a sample of tissue, such as, for example, the specimen handling tool 24 such as that shown in Figures 5 and 10-15, may also be included in particular embodiments of the diagnostic system 20 of the present invention. The specimen-handling tool 24 may be disposed within the cavity 30.

The cavity 30 may, as shown in Figures 5-7, be configured so that it is disposed about at least a portion of one of the first and/or second wells. 26 and 28, respectively. The container 22 may also be configured so that a specimen handling tool 24 may be otherwise retained in the container 22 so that it is disposed about at least a portion of one of the first and/or second wells 26 and 28, respectively. The specimen handling tool can be shaped like a pair of tweezers as shown or in the shape of a single member pointed instrument that can pick up a specimen by lancing the sample. As shown in Figures 16 and 17, the container 22 may be configured so that the specimen-handling tool 24 is secured in a particular position by one or more ribs 84. The specimen-handling tool 24 may be removably attached to the container 22 by one or more locking arms, breakaway tabs, adhesive, or the like.

One or more rails 46 may be included in selected embodiments of the present invention and may be disposed on the container 22 so that the rails extend upwardly along at least a portion of the surface 44. One or more rails 46 may also be configured to extend outwardly from the container 22. At least one gap 48 may be formed in one of the rails 46 that extend along a portion of the container 22.

As shown in Figure 7, one or more supports 50 may be provided which extend downwardly from the surface 44. As seen in Figure 7, the supports 50 may be attached to the wall (or walls) 31 that form at least a portion of the cavity 30. The supports 50 may extend outwardly from the wall 31 to permit the container 22 to rest in a stable position on a horizontal or other surface. The rails 46 and the supports 50 may be configured to enable the container 22 to be automatically processed through a variety of equipment.

If desired, the surface 44 may be configured so that various indicia, such as letters, numbers, symbols and other characters, may be placed onto or formed into the surface 44. For example, and as shown in Figure 6, each well 26 and/or 28 may be given a particular designation, such as A or B, and that designation may be printed upon the surface 44.

The container 22 may be formed from a variety of materials, including, for example, polycarbonate, polystyrene, polypropylene, polyethylene, polyvinylchloride, or any other type of polyolefin.

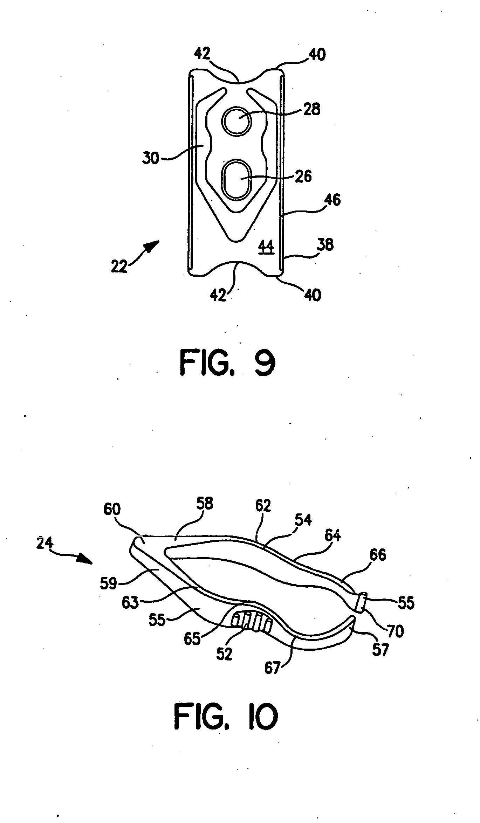

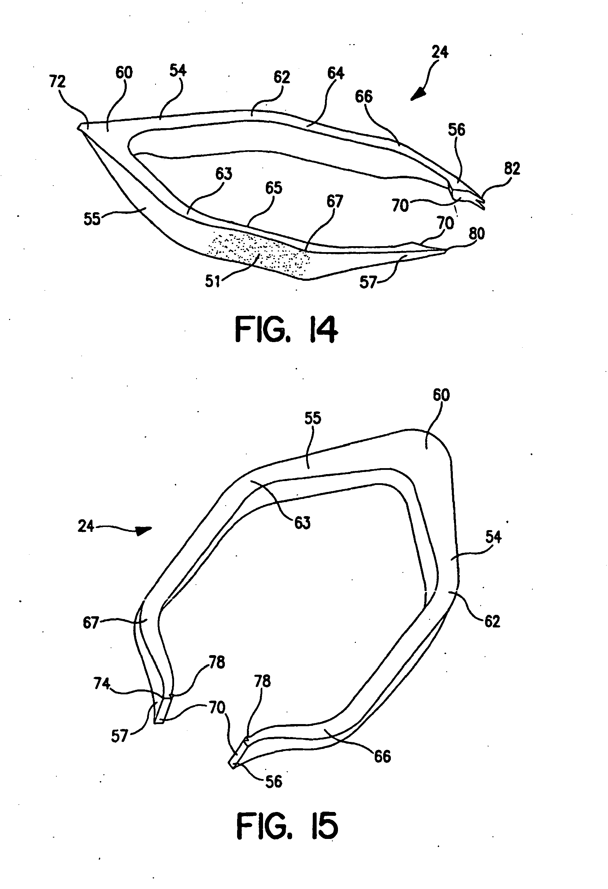

Particular embodiments of the specimen-handling tool 24 are shown in Figures 10-15 and 21. The specimen-handling tool 24 may include, as shown in Figures 10-13, a pair of cooperating arms 54 and 55. Each arm 54 and 55 may include a tip portion 56 and 57, respectively. The arms 54 and 55 may each also include a rear portion 58 and 59, respectively. The arms 54 and 55 may be joined to each other at their rear portions 58 and 59, respectively, forming a joined end 60. The joined end 60 may be configured to assist the user in accomplishing particular tasks, such as, for example, manipulating a specimen, removing a plug 86 from one of the first and/or second wells 26 and 28, respectively, as well as other tasks. The outermost portion of the joined end 60 may be variously configured, and may be formed as a narrow projection, such as that shown in Figure 14.

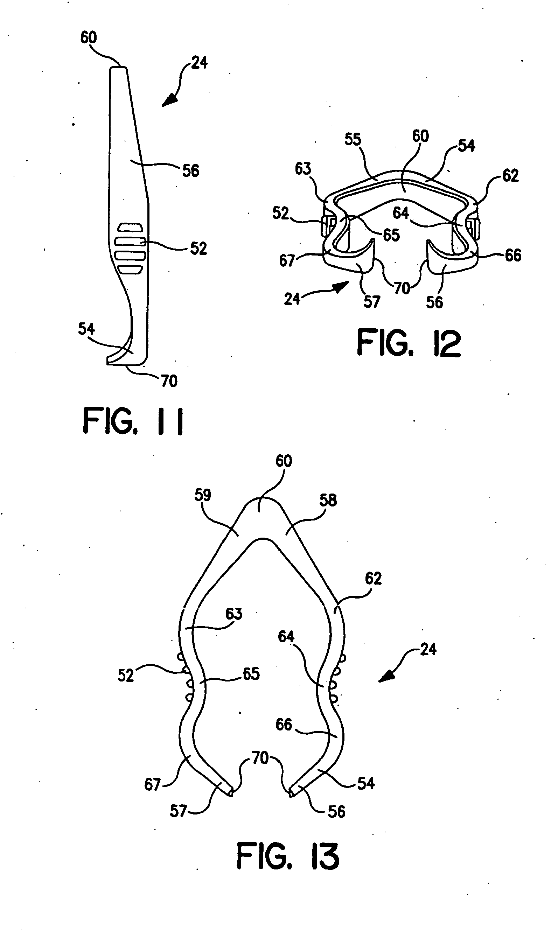

As seen in Figures 12 and 13, each arm 54 and 55 may also include a rearward arcuate portion 62 and 63, respectively, and a forward arcuate portion 66 and 67, respectively. Disposed between each rearward arcuate portion 62 and 63 and its corresponding forward arcuate portion 66 and 67, respectively, is an intermediate arcuate portion 64 and 65, respectively. The arcuate portions 62-64-66 and 63-65-67 of each arm 54 and 55, respectively, may be configured so that the area disposed between the arms 54 and 55 is approximately hourglass in shape. In such an embodiment, the rearward arcuate portions 62 and 63 and forward arcuate portions 66 and 67 curve outwardly, and the intermediate arcuate portions 64 and 65 curve inwardly.

The intermediate arcuate portions 64 and 65 may be formed so that a user may more easily grip these portions. As shown in Figure 10, one or more ribs 52 may be positioned on the outer surface of the intermediate arcuate portions 64 and 65. Alternately, a portion of the arms 54 and/or 55 may have a roughened texture to enable a user to more effectively grasp and manipulate the specimen-handling tool 24, such as is shown in Figure 14 at 51.

The arms 54 and/or 55 may include fewer or more arcuate portions than the three arcuate portions described above, such as the specimen-handling tool shown in Figure 15. The arcuate portions of the arms 54 and/or 55 may have a more or less pronounced arcuate shape than what is depicted in Figure 10. For example and as shown in Figures 14 - 16 and 21, other configurations of the arms 54 and 55 may be used in the specimen-handling tool 24.

The tip portions 56 and 57 may be variously formed to enable a user to manipulate a specimen. The tip portions 56 and 57 may be formed to include a surface such as the surfaces 70. The surfaces 70 may be variously shaped and, in particular, one or both of the surfaces 70 may be curved (as shown in Figure 14) or flat (as shown in Figure 10). The surfaces 70 may be rough or smooth. Also, structures such as the ridges 78 that are depicted in Figure 15 may also be positioned on one or more of the surfaces 70. The surfaces 70 may be disposed so that they are at least somewhat facing each other, thereby enabling a user to grasp a specimen and hold it between the surfaces 70. As shown in Figure 14, the tip portions 56 and/or 57 may curve outwardly, and may, in some embodiments such as is shown in Figure 15, end in a relatively sharp edge 74. One or both of the tip portions 56 and 57 may include a point, such as the point 80 shown in Figure 14 or a fork 82, also shown in Figure 14, or any number of other configurations.

The specimen-handling tool may be formed from a variety of materials, including, for example, polycarbonate, polystyrene, polypropylene, polyethylene, polyvinylchloride, or any other type of polyolefin.

Referring now to Figures 19 and 20, an overlying member 23 may be disposed over at least a portion of the surface 44 of the container 22. At least a portion of the cavity 30 may be formed by the wall 31. The overlying member 23 may take the form of an adhesive-backed label that adheres to at least a portion of the surface 44. The overlying member 23 may overly any combination of the first well 26, the second well 28 and the cavity 30.

The overlying member 23 may also be used to seal the first and second wells 26 and 28, respectively. In some embodiments, the overlying member may be used to regulate the rate of water vapor transmission to and from the wells 26 and 28 of the container 22. The overlying member 23 may also be configured so that, if the overlying member 23 is removed prematurely or inadvertently, it may be easily reapplied to the container 22 so that the wells 26 and 28 may be resealed.

The overlying member 23 may also be used to retain the specimen-handling tool 24 within the cavity 30. The overlying member 23 may also be configured only to retain the specimen-handling tool 24 within the cavity 30. In some embodiments, the overlying member 23 may be adhered to at least a portion of the specimen-handling tool 24 so that, when the overlying member 23 is removed form the container 22, the specimen-handling tool 24 is also removed from the container 22. Although this may be accomplished in many different ways, the intermediate arcuate portions 64 and 65 may, when the specimen-handling tool 24 is positioned within the cavity 30, be level with or rise slightly above the surface 44 so as to contact and be adhered to the overlying member 23.

As shown in Figure 20, a plug 87 may also be used to at least partially seal each well 26 and 28. In such a configuration, the overlying member 23 does not need to seal the well that contains the plug 87, but may merely be positioned above the well 26 and/or 28. The plug 87 may be formed from a variety of materials, including, for example, rubber, wax, silicone, or any of a variety of plastics. In some embodiments, a film cover 86, shown in Figure 18, may also be applied to a portion of the container 22, such as, for example, the well 28.

In some embodiments, the overlying member 23 may be adhered or otherwise connected to one or more of the plugs 87 so that, when the overlying member 23 is separated from the container 22, one or more of the plugs 87 may also be removed. The plug 87 may also be removed with the specimen-handling tool.

### Example

The following example was performed in order to demonstrate the stability of a urease testing device made in accordance with the present invention.

A test slide according to the present invention was prepared containing the urea composition and the indicator gel composition described above. The indicator gel composition, however, did not contain the methyl paraben bactericide or the citric acid pH adjuster.

Specifically, the gel composition contained the following:

| **Ingredient** | **Amount** |
|---|---|
| Extra Pure Grade Agar | 1.4941g |
| Phenol Red | 0.0110g |
| Distilled Water | 100.00mL |

The shelf life of the above prepared slide was then compared with the shelf life of a commercial product marketed under the name CLO-TEST by Ballard Medical/Kimberly Clark of Draper, Utah. The CLO-TEST product includes a urease indicator composition which contains a mixture of urea and an indicator in a gel as described in U.S. Patent No. 4,748,113.

Three test slides made according to the present invention were compared with three samples of the CLO-TEST product. A standardized CLO-TEST Color Chart developed prior to the experiment was used to assign numerical scores to the color of the samples during the experiment.

The slides were affixed to a polystyrene box introduced into a chamber set at 37°C, 100% relative humidity, and 10% carbon dioxide. Photographs were taken every 24 hours for a period of 45 days, which were then assessed and given a score using the CLO-TEST Color Chart. Using color readings with scores of equal to or greater than 4 as unusable, the CLO-TEST samples were deemed unusable on day 4, while the test slides of the present invention were still viable on day 45.

The shelf life of the test slide of the present invention was also tested with an artificial biopsy by means of a tissue sample containing deliberately introduced urease. The artificial biopsy sample was placed in the first well containing the powdered urea. The sample was coated with urea, and then placed in the second well containing the indicator gel composition. Observations of the color change of the gel revealed it was still viable for the detection of ammonia after 39 days, when the gel was checked.

It should be noted that any given range presented herein is intended to include any and all lesser included ranges. For example, a range of from 45-90 would also include 50-90; 45-80; 46-89 and the like. Thus, the range of 95% to 99.999% also includes, for example, the ranges of 96% to 99.1 %, 96.3% to 99.7%, and 99.91 to 99.999%.

## Claims

1. A method for detecting the presence of urease In a gastrointestinal system comprising:
on a sample of gastric material obtained from a patient;
contacting said gastric material with a first composition comprising urea, said urea being capable of being converted into ammonia when contacted with urease; and
thereafter contacting said gastric material with a second composition comprising an indicator, said indicator being configured to indicate the presence of ammonia thereby indicating the presence of urease in said gastric material.

2. A method as defined in claim 1, wherein the first composition comprises a powdered composition.

3. A method as defined in claim 2, wherein said urea has a mean particle size of less than 0.1 mm.

4. A method as defined in claim 2, wherein said first composition further comprises an anti-caking agent.

5. A method as defined in claim 1, wherein said second composition comprises agar in addition to said indicator.

6. A method as defined in claim 1, wherein said indicator comprises a pH indicator that changes color when the pH is Increased.

7. A method as defined in claim 1, wherein said second composition further comprises a bactericide or a bacteristat.

8. A method as defined in claim 1, wherein said second composition further comprises a pH adjuster.

9. A method as defined in claim 1, wherein said gastric material is contacted with said first composition such that at least a portion of the urea is combined with the gastric material prior to being contacted with said second composition.

10. A method as defined in claim 1, wherein the second composition comprises a gel.

11. A method as defined in claim 1, wherein the indicator comprises phenol red.

12. A method for detecting the presence of urease in a gastrointestinal system comprising:
on a sample of gastric material obtained from a patient;
contacting said gastric material with a composition comprising a powdered urea and sequentially a dry indicator, said urea being capable of being converted into ammonia when contacted with urease and said indicator being configured to indicate the presence of ammonia thereby indicating the presence of urease in said gastric material.

13. A method as defined in claim 12, wherein said urea present within said composition has a mean particle size of less than about 0.1 mm.

14. A method as defined in claim 12, wherein said composition further comprises an anti-caking agent.

15. A method as defined in claim 12, wherein said indicator comprises a pH indicator that changes color when the pH is increased.

16. A system for detecting the presence of urease comprising:
a first composition separated from a second composition for sequential contact with a sample, said first composition comprising urea in powdered form, said urea being capable of being converted into ammonia when contacted with urease, said second composition comprising an indicator, said indicator being configured to indicate the presence of ammonia.

17. A system as defined in claim 16, wherein said second composition comprises a gel.

18. A system as defined in claim 16, wherein said second composition further comprises agar and a pH adjuster, said second composition having a pH of less than about 6.0.

19. A system as defined in claim 16, wherein said first composition is contained in a first container and said second composition is contained in a second container.

20. A system as defined in claim 16, wherein said first composition and said second composition are positioned, and spaced apart, in the same container.

21. A system as defined in claim 16, wherein the urea has a particle size of less than about 0.1 mm.

22. A system for detecting the presence of urease comprising:
a container including a first well spaced apart from a second well;
a first composition contained in said first well, said first composition comprising urea, said urea being capable of being converted into ammonia when contacted with urease;
a second composition contained in said second well, said second composition comprising an indicator, said indicator being configured to indicate the presence of ammonia.

23. A system as defined In claim 22 wherein said first composition comprises a powder and wherein said urea has a mean particle size of less than about 0.1 mm.

24. A system as defined In claim 23, wherein said second composition further comprises agar and a pH adjuster.

25. A system as defined in claim 22, wherein said indicator comprises phenol red.

26. A system as defined in claim 22, further comprising a top covering said first well and said second well of said container, said top covering comprising a film, said film being water impermeable over said first well.

27. A system as defined in claim 26 further comprising a removable plug disposed In at least one of the wells.

28. A system as defined in claim 22, further comprising a specimen-handling tool.

29. A system as defined in claim 28, wherein said specimen-handling tool is disposed within at least a portion of said container.

30. A system as defined in claim 28. the specimen-handling tool comprising a pair of cooperating arms.

31. A system as defined in claim 30, each arm of the specimen handling tool comprising a tip portion and a rear portion, the arms being joined to each other at their rear portions to form a joined end.

32. A system as defined in claim 31, at least one tip portion being formed as a flat surface.

33. A system as defined in claim 31, the joined end being formed to include a narrow projection.

34. A system as defined in claim 30, each arm further comprising a rearward arcuate portion.

35. A system as defined in claim 30, each arm further comprising a forward arcuate portion.

36. A system as defined in claim 35, each arm further comprising a rearward arcuate portion and an intermediate arcuate portion, the intermediate arcuate portion being disposed between the rearward arcuate portion and the forward arcuate portion.

37. A system as defined in claim 22, wherein at least one of the wells has a frustoconical configuration.

## Patentansprüche

1. Ein Verfahren zum Detektieren des Vorhandenseins von Urease in einem gastrointestinalen System, das umfasst:
auf einer Probe von gastrischem Material, die von einem Patienten erhalten wurde
Kontaktieren des genannten gastrischen Materials mit einer ersten Zusammensetzung, die Harnstoff umfasst, wobei der Harnstoff dazu geeignet ist, in Ammoniak umgewandelt zu werden, wenn er mit Urease in Kontakt gerät; und
danach Kontaktieren des genannten gastrischen Materials mit einer zweiten Zusammensetzung, die einen Indikator umfasst, wobei der genannte Indikator dazu ausgebildet ist, das Vorhandensein von Ammoniak anzuzeigen, wodurch das Vorhandensein von Urease in dem genannten gastrischen Material angezeigt wird.

2. Ein Verfahren, wie es in Anspruch 1 definiert ist, in dem die genannte erste Zusammensetzung eine pulverisierte Zusammensetzung umfasst.

3. Ein Verfahren, wie es in Anspruch 2 definiert ist, in dem der genannte Harnstoff eine mittlere Teilchengröße von weniger als 0,1 mm aufweist.

4. Ein Verfahren, wie es in Anspruch 2 definiert ist, in dem die genannte erste Zusammensetzung weiterhin ein Antiagglomerationsmittel umfasst.

5. Ein Verfahren, wie es in Anspruch 1 definiert ist, in dem die genannte zweite Zusammensetzung zusätzlich zu dem genannten Indikator Agar umfasst.

6. Ein Verfahren, wie es in Anspruch 1 definiert ist, in dem der genannte Indikator einen pH - Indikator umfasst, der die Farbe ändert, wenn der pH - Wert erhöht wird.

7. Ein Verfahren, wie es in Anspruch 1 definiert ist, in dem die genannte zweite Zusammensetzung weiterhin ein Bakterizid oder ein Bakteriostat umfasst.

8. Ein Verfahren, wie es in Anspruch 1 definiert ist, in dem die genannte zweite Zusammensetzung weiterhin ein pH - Anpassungsmittel umfasst.

9. Ein Verfahren, wie es in Anspruch 1 definiert ist, in dem das genannte gastrische Material mit der genannten ersten Zusammensetzung kontaktiert wird, so dass zumindest ein Teil des Harnstoffs mit dem gastrischen Material kombiniert wird, bevor er mit der zweiten Zusammensetzung kontaktiert wird.

10. Ein Verfahren, wie es in Anspruch 1 definiert ist, in dem die zweite Zusammensetzung ein Gel umfasst.

11. Ein Verfahren, wie es in Anspruch 1 definiert ist, in dem der Indikator Phenolrot umfasst.

12. Ein Verfahren zum Detektieren des Vorhandenseins von Urease in einem gastrointestinalen System, das umfasst:
auf einer Probe von gastrischem Material, die von einem Patienten erhalten wurde
Kontaktieren des genannten gastrischen Materials mit einer Zusammensetzung, die einen pulverisierten Harnstoff umfasst, und danach mit einem trockenen Indikator, wobei der genannte Harnstoff dazu geeignet ist, in Ammoniak umgewandelt zu werden, wenn er mit Urease in Kontakt gerät, und der genannte Indikator dazu ausgebildet ist, das Vorhandensein von Ammoniak anzuzeigen, wodurch das Vorhandensein von Urease in dem genannten gastrischen Material angezeigt wird.

13. Ein Verfahren, wie es in Anspruch 12 definiert ist, in dem der genannte Harnstoff, der in der genannten Zusammensetzung vorhanden ist, eine mittlere Teilchengrö-ße von weniger als 0,1 mm aufweist.

14. Ein Verfahren, wie es in Anspruch 12 definiert ist, in dem die genannte Zusammensetzung weiterhin ein Antiagglomerationsmittel umfasst.

15. Ein Verfahren, wie es in Anspruch 12 definiert ist, in dem der genannte Indikator einen pH - Indikator umfasst, der die Farbe ändert, wenn der pH - Wert erhöht wird.

16. Ein System zum Detektieren des Vorhandenseins von Urease, das umfasst:
eine erste Zusammensetzung, die von einer zweiten Zusammensetzung für das aufeinanderfolgende Kontaktieren mit einer Probe getrennt ist, wobei die erste Zusammensetzung Harnstoff in pulverisierter Form umfasst, wobei der genannte Harnstoff dazu geeignet ist, in Ammoniak umgewandelt zu werden, wenn er mit Urease in Kontakt gerät, wobei die zweite Zusammensetzung einen Indikator umfasst, wobei der genannte Indikator dazu ausgebildet ist, das Vorhandensein von Ammoniak anzuzeigen.

17. Ein System, wie es in Anspruch 16 definiert ist, in dem die zweite Zusammensetzung ein Gel umfasst.

18. Ein System, wie es in Anspruch 16 definiert ist, in dem die zweite Zusammensetzung weiterhin Agar und ein pH - Anpassungsmittel umfasst, wobei die zweite Zusammensetzung einen pH - Wert von weniger als 6,0 aufweist.

19. Ein System, wie es in Anspruch 16 definiert ist, in dem die genannte erste Zusammensetzung in einem ersten Behälter enthalten ist, und die genannte zweite Zusammensetzung in einem zweiten Behälter enthalten ist.

20. Ein System, wie es in Anspruch 16 definiert ist, in dem sich die genannte erste Zusammensetzung und die genannte zweite Zusammensetzung in demselben Behälter und voneinander räumlich getrennt befinden.

21. Ein System, wie es in Anspruch 16 definiert ist, in dem die Urease eine Teilchengröße von weniger als ungefähr 0,1 mm aufweist.

22. Ein System zum Detektieren des Vorhandenseins von Urease, das umfasst:
einen Behälter, der einen ersten Schacht beabstandet von einem zweite Schacht einschließt;
eine erste Zusammensetzung, die in dem genannten ersten Schacht enthalten ist, wobei die genannte erste Zusammensetzung einen Harnstoff umfasst, wobei der genannte Harnstoff dazu geeignet ist, in Ammoniak umgewandelt zu werden, wenn er mit Urease in Kontakt gerät;
eine zweite Zusammensetzung, die in dem genannten zweiten Schacht enthalten ist, wobei die genannte zweite Zusammensetzung einen Indikator umfasst, der dazu ausgebildet ist, das Vorhandensein von Ammoniak anzuzeigen.

23. Ein System, wie es in Anspruch 22 definiert ist, in dem die genannte erste Zusammensetzung ein Pulver umfasst, und in dem der genannte Harnstoff eine mittlere Teilchengröße von weniger als 0,1 mm aufweist.

24. Ein System, wie es in Anspruch 23 definiert ist, in dem die genannte zweite Zusammensetzung weiterhin Agar und ein pH - Anpassungsmittel umfasst.

25. Ein System, wie es in Anspruch 22 definiert ist, in dem der genannte Indikator Phenolrot umfasst.

26. Ein System, wie es in Anspruch 22 definiert ist, das weiterhin eine obere Abdeckung des genannten ersten Schachts und des genannten zweiten Schachts des genannten Behälters umfasst, wobei die genannte obere Abdeckung einen Film umfasst, wobei der genannte Film über dem genannten ersten Schacht wasserundurchlässig ist.

27. Ein System, wie es in Anspruch 26 definiert ist, das weiterhin einen entfernbaren Stopfen umfasst, der in zumindest einem der Schächte angeordnet ist.

28. Ein System, wie es in Anspruch 22 definiert ist, das weiterhin ein Probenhandhabungsmittel umfasst.

29. Ein System, wie es in Anspruch 28 definiert ist, in dem das Probenhandhabungsmittel zumindest in einem Teil des genannten Behälters angeordnet ist.

30. Ein System, wie es in Anspruch 28 definiert ist, in dem das Probenhandhabungsmittel ein Paar von zusammenwirkenden Armen umfasst.

31. Ein System, wie es in Anspruch 30 definiert ist, wobei jeder Arm des Probenhandhabungsmittels einen vorderen Bereich und einen hinteren Bereich umfasst, wobei die Arme an ihren hinteren Bereichen verbunden sind, so dass ein verbundenes Ende gebildet wird.

32. Ein System, wie es in Anspruch 31 definiert ist, in dem zumindest ein vorderer Bereich als eine flache Fläche ausgebildet ist.

33. Ein System, wie es in Anspruch 31 definiert ist, in dem das verbundene Ende so ausgebildet ist, dass es einen schmalen Vorsprung einschließt.

34. Ein System, wie es in Anspruch 30 definiert ist, in dem jeder Arm weiterhin einen hinteren gebogenen Bereich umfasst.

35. Ein System, wie es in Anspruch 30 definiert ist, in dem jeder Arm weiterhin einen vorderen gebogenen Bereich umfasst.

36. Ein System, wie es in Anspruch 35 definiert ist, in dem jeder Arm weiterhin einen hinteren gebogenen Bereich und einen mittleren gebogenen Bereich umfasst, wobei der mittlere gebogene Bereich zwischen dem hinteren gebogenen Bereich und dem vorderen gebogenen Bereich angeordnet ist.

37. Ein System, wie es in Anspruch 22 definiert ist, in dem zumindest einer der Schächte einen kegelstumpfartigen Aufbau besitzt.

## Revendications

1. Procédé pour la détection de la présence d'uréase dans un système gastro-intestinal comprenant:
sur un échantillon d'un matériau gastrique obtenu d'un patient ;
la mise en contact dudit matériau gastrique avec une première composition comprenant de l'urée, ladite urée étant capable d'être convertie en ammoniac lors d'une mise en contact avec une uréase ; et
par la suite, la mise en contact dudit matériau gastrique avec une seconde composition comprenant un indicateur, ledit indicateur étant réalisé pour indiquer la présence d'ammoniac, en indiquant ainsi la présence d'uréase dans ledit matériau gastrique.

2. Procédé selon la revendication 1, dans lequel la première composition comprend une composition pulvérulente.

3. Procédé selon la revendication 2, dans lequel ladite urée a une granulométrie moyenne inférieure à 0,1 mm.

4. Procédé selon la revendication 2, dans lequel ladite première composition comprend en outre un agent anti-agglutinant.

5. Procédé selon la revendication 1, dans lequel ladite seconde composition comprend de la gélose en plus dudit indicateur.

6. Procédé selon la revendication 1, dans lequel ledit indicateur comprend un indicateur de pH qui change de couleur quand le pH est augmenté.

7. Procédé selon la revendication 1, dans lequel ladite seconde composition comprend en outre un bactéricide ou un bactériostatique.

8. Procédé selon la revendication 1, dans lequel ladite seconde composition comprend en outre un ajusteur de pH.

9. Procédé selon la revendication 1, dans lequel ledit matériau gastrique est mis en contact avec ladite première composition de sorte qu'au moins une partie de l'urée soit combinée avec le matériau gastrique avant la mise en contact avec ladite seconde composition.

10. Procédé selon la revendication 1, dans lequel la seconde composition comprend un gel.

11. Procédé selon la revendication 1, dans lequel l'indicateur comprend du rouge de phénol.

12. Procédé pour la détection de la présence d'uréase dans un système gastro-intestinal comprenant :
sur un échantillon de matériau gastrique obtenu d'un patient ;
la mise en contact dudit matériau gastrique avec une composition comprenant une urée en poudre et consécutivement un indicateur sec, ladite urée étant capable d'être convertie en ammoniac lors d'une mise en contact avec de l'uréase et ledit indicateur étant réalisé pour indiquer la présence d'ammoniac, en indiquant ainsi la présence d'uréase dans ledit matériau gastrique.

13. Procédé selon la revendication 12, dans lequel ladite urée présente dans ladite composition a une granulométrie moyenne inférieure à environ 0,1 mm.

14. Procédé selon la revendication 12, dans lequel ladite composition comprend en outre un agent anti-agglutinant.

15. Procédé selon la revendication 12, dans lequel ledit indicateur comprend un indicateur de pH qui change de couleur quand le pH est augmenté.

16. Système pour la détection de la présence d'uréase comprenant :
une première composition séparée d'une seconde composition pour un contact consécutif avec un échantillon, ladite première composition comprenant de l'urée sous forme pulvérulente, ladite urée étant capable d'être convertie en ammoniac lors d'une mise en contact avec de l'uréase, ladite seconde composition comprenant un indicateur, ledit indicateur étant réalisé pour indiquer la présence d'ammoniac.

17. Système selon la revendication 16, dans lequel ladite seconde composition comprend un gel.

18. Système selon la revendication 16, dans lequel ladite seconde composition comprend en outre de la gélose et un ajusteur de pH, ladite seconde composition ayant un pH inférieur à environ 6,0.

19. Système selon la revendication 16, dans lequel ladite première composition est contenue dans un premier récipient et ladite seconde composition est contenue dans un second récipient.

20. Système selon la revendication 16, dans lequel ladite première composition et ladite seconde composition sont positionnées, et espacées, dans le même récipient.

21. Système selon la revendication 16, dans lequel l'urée a une granulométrie inférieure à environ 0,1 mm.

22. Système pour la détection de la présence d'uréase comprenant:
un récipient comportant un premier puits espacé d'un second puits ;
une première composition contenue dans ledit premier puits, ladite première composition comprenant de l'urée, ladite urée étant capable d'être convertie en ammoniac lors d'une mise en contact avec de l'uréase ;
une seconde composition contenue dans ledit second puits, ladite seconde composition comprenant un indicateur, ledit indicateur étant réalisé pour indiquer la présence d'ammoniac.

23. Système selon la revendication 22, dans lequel ladite première composition comprend une poudre et dans lequel ladite urée a une granulométrie moyenne inférieure à environ 0,1 mm.

24. Système selon la revendication 23, dans lequel ladite seconde composition comprend en outre de la gélose et un ajusteur de pH.

25. Système selon la revendication 22, dans lequel ledit indicateur comprend du rouge de phénol.

26. Système selon la revendication 22, comprenant en outre une partie supérieure recouvrant ledit premier puits et ledit second puits dudit récipient, ladite partie supérieure recouvrant comprenant un film, ledit film étant imperméable à l'eau au-dessus dudit premier puits.

27. Système selon la revendication 26, comprenant en outre un bouchon retirable disposé dans au moins un des puits.

28. Système selon la revendication 22, comprenant en outre un outil de manipulation des spécimens.

29. Système selon la revendication 28, dans lequel ledit outil de manipulation des spécimens est disposé dans au moins une partie dudit récipient.

30. Système selon la revendication 28, l'outil de manipulation des spécimens comprenant une paire de bras en coopération.

31. Système selon la revendication 30, chaque bras de l'outil de manipulation des spécimens comprenant une partie de tête et une partie arrière, les bras étant joints l'un à l'autre au niveau de leur partie arrière pour former une extrémité jointe.

32. Système selon la revendication 31, au moins une partie de tête étant formée en tant que surface plane.

33. Système selon la revendication 31, l'extrémité jointe étant formée de manière à inclure une prolongation étroite.

34. Système selon la revendication 30, chaque bras comprenant en outre une partie arquée vers l'arrière.

35. Système selon la revendication 30, chaque bras comprenant en outre une partie arquée vers l'avant.

36. Système selon la revendication 35, chaque bras comprenant en outre une partie arquée vers l'arrière et une partie arquée intermédiaire, la partie arquée intermédiaire étant disposée entre la partie arquée vers l'arrière et la partie arquée vers l'avant.

37. Système selon la revendication 22, dans lequel au moins un des puits a une configuration tronconique.
